# EUROPÄISCHE PATENTSCHRIFT

(11) **EP 3 044 304 B1**
(45) Veröffentlichungstag und Bekanntmachung des Hinweises auf die Patenterteilung: **12.07.2017**
(21) Anmeldenummer: 14758796.8
(22) Anmeldetag: 18.08.2014
(51) Int. Cl.: C12M 1/00, C12M 1/26, C12M 1/12

(54) **BEHÄLTER MIT FLEXIBLER WANDUNG**
CONTAINER WITH A FLEXIBLE WALL
CONTENEUR À PAROI FLEXIBLE

(30) Priorität: 09.09.2013 DE 102013109819
(43) Veröffentlichungstag der Anmeldung: 20.07.2016
(73) Patentinhaber: Sartorius Stedim Biotech GmbH, 37079 Göttingen (DE)
(72) Erfinder: HUSEMANN, Bernward, 37079 Göttingen (DE); GRELLER, Gerhard, 37085 Göttingen (DE); KAHLERT, Wolfgang, 34327 Körle (DE); KEITEL, Heinz-Rüdiger, verstorben (DE); DE WILDE, Davy, 4140 Sprimont (BE)
(74) Vertreter: Schneider, Peter Christian
(86) Internationale Anmeldenummer: PCT/EP2014/067582
(87) Internationale Veröffentlichungsnummer: WO 2015/032612

(56) Entgegenhaltungen:
- WO-A1-2007/044047
- WO-A1-2011/082787
- WO-A2-2007/134267
- WO-A2-2009/122310
- WO-A2-2011/094534
- DE-A1-102006 014 495
- DE-A1-102008 010 427
- DE-A1-102009 037 345
- DE-U1-202012 005 989
- US-A1- 2002 015 671
- US-A1- 2011 038 222
- US-A1- 2012 077 243

## Beschreibung

### Gebiet der Erfindung

Die Erfindung betrifft einen Behälter mit flexibler Wandung und mindestens einem durch einen Wandungsdurchbruch in den von der flexiblen Wandung umgebenen Behälterinnenraum hineinragenden Entleerungsrohr, dessen freies Ende eine Entleerungsöffnung aufweist, die in ihrer Lage auf unterschiedliche Entleerungshöhen in vertikaler Richtung einstellbar ist.

### Stand der Technik

Aus der WO 2011/094534 A2 ist ein Behälter mit flexibler Wandung bekannt, der einen Behälterinnenraum aufweist, in den ein Entleerungsrohr mit seinem freien Ende durch einen Wandungsdurchbruch hineinragt. Das freie Ende des Entleerungsrohres weist eine Entleerungsöffnung auf, die auf unterschiedliche Entleerungshöhen einstellbar ist.

Nachteilig dabei ist, dass das Entleerungsrohr durch einen an einer Seitenwandung in einem Wandungsdurchbruch angeordneten Drehadapter in den Behälterinnenraum hineinragt und im Behälterinnenraum das freie Ende um 90° abgewinkelt sein muss. Das Entleerungsrohr ist drehbar in dem Drehadapter gelagert, wobei durch eine Drehung die Entleerungshöhe verändert werden kann. Falls bei einem zylindrisch ausgebildeten Behälter das abgewinkelte freie Ende des Entleerungsrohres nah an der Wandung platziert ist, kann es an der Wandung anstoßen und kann dann nicht um 360° gedreht werden. Bei einem Einbau in der Nähe der Behältermitte kann das freie Ende an einen Rührer in der Behältermitte anstoßen. Weiterhin nachteilig ist, dass das drehbar in dem Adapter geführte Entleerungsrohr nur sehr schwer abzudichten ist. Wegen der damit verbundenen relativ hohen Kosten ist der bekannte Behälter somit nicht als wirtschaftlicher Einwegbehälter geeignet.

Aus der US 2012/077243 A1 ist ein Behälter mit flexibler Wandung und mindestens einem durch einen Wandungsdurchbruch in den von der flexiblen Wandung umgebenen Behälterinnenraum hineinragenden Entleerungsrohr, dessen freies Ende eine Entleerungsöffnung aufweist, bekannt.

Nachteilig bei dem bekannten Behälter ist, dass das Entleerungsrohr im Bereich des Wandungsdurchbruches des Behälters verschweißt ist. Dies hat zwar den Vorteil, dass das Entleerungsrohr durch die Verschweißung fluiddicht in dem Wandungsdurchbruch angeordnet ist, wird aber durch den Nachteil erkauft, dass das Entleerungsrohr nach dem Verschweißen seines freien Endes nicht mehr auf unterschiedliche Entleerungshöhen in vertikaler Richtung einstellbar ist.

Aus der WO 2011/082787 A1 ist ein Behälter mit flexibler Wandung und einem Wandungsdurchbruch, durch den eine Komponente aus dem Behälter entnommen werden kann, bekannt. Ein Rührwerksmast kann dabei mit seinem freien Ende aus dem Wandungsdurchbruch herausragen, wobei das freie Ende von einer flexiblen Umhüllung umgeben ist, die an einem ersten Ende durch eine Verschlussvorrichtung verschlossen ist und an ihrem dem ersten Ende abgewandten zweiten Ende mit der den Wandungsdurchbruch umgebenden Wandung fluiddicht verbunden ist.

Nachteilig bei der aus der WO 2011/082787 A1 bekannten Vorrichtung ist, dass zur Entnahme der Komponente zum einen eine zusätzliche Abschnüreinrichtung im Bereich des zweiten Endes und zum anderen die Verschlussvorrichtung im Bereich des ersten Endes notwendig sind. Nachteilig ist zudem, dass wenn auch nur geringfügig, Gas aus dem Behälterinnenraum entweichen kann.

Aus der US 2011/038222 A1 ist ein Mischer-Behälter bekannt, dessen schlauchförmige Zuführelemente für Belüftungsgas mittels Festhalteteilen an der Wand festgehalten werden. Eine Längsbewegung der Zuführelemente im Sinne einer Schlauchführung ist dabei jedoch nicht vorgesehen und im Sinne einer Fixierung unerwünscht.

### Aufgabenstellung

Es ist die Aufgabe der vorliegenden Erfindung, einen Behälter mit flexibler Wandung anzugeben, der ein Entleerungsrohr aufweist, wobei die Entleerungsöffnung auf unterschiedliche Entleerungshöhen einstellbar sein soll. Dabei soll das Entleerungsrohr gegenüber dem Behälter sicher und kostengünstig ausgebildet sein, so dass der Behälter insbesondere auch geeignet ist, um als Einwegbehälter verwendet zu werden.

### Darlegung der Erfindung

Diese Aufgabe wird in Verbindung mit den Merkmalen des Oberbegriffs von Anspruch 1 dadurch gelöst, dass in dem Wandungsdurchbruch ein Adapterstück angeordnet ist, in dem das Entleerungsrohr längsverschieblich gehaltert ist, dass das Entleerungsrohr in einem Teilbereich von einer flexiblen Umhüllung umgeben ist, die an einem ersten Ende mit dem Entleerungsrohr und an ihrem dem ersten Ende abgewandten zweiten Ende mit der Wandung oder dem Adapterstück fluiddicht verbunden ist, und dass die flexible Umhüllung im Behälterinnenraum angeordnet ist.

Durch die flexible Umhüllung, die an einem Ende mit der Wandung des flexiblen Behälters oder mit dem Adapterstück und am anderen Ende mit dem Entleerungsrohr dicht verbunden ist, wird eine einfache und sichere Abdichtung ohne Kontaminationsgefahr erreicht. Die flexible Umhüllung bildet eine Sterilbarriere. Die Anordnung der flexiblen Umhüllung im Behälterinnenraum ermöglicht, die Umhüllung relativ dünn auszubilden, da sie im Behälterinnenraum von der Wandung des Behälters geschützt wird. Durch die sichere Abdichtung über die flexible Umhüllung kann das Entleerungsrohr relativ einfach, beispielsweise über O-Ringdichtungen, verschieblich in dem Adapterstück geführt und befestigt werden. Das Adapterstück kann somit ebenfalls einfach und kostengünstig ausgebildet sein. Damit ist der Behälter insbesondere auch als kostengünstiger und sicherer Einwegbehälter geeignet. Das in den Behälterinnenraum hineinragende Entleerungsrohr kann beispielsweise vertikal von oben oder auch seitlich, schräg in den Behälterinnenraum hineinragen.

Anstatt der flexiblen Umhüllung können auch O-Ringdichtungen oder ähnliche Dichtungselemente eingesetzt werden, welche insbesondere für die Verwendung in Glas-oder Edelstahlbehältern geeignet sind.

Bevorzugte Ausführungsformen sind Gegenstand der abhängigen Ansprüche.

Gemäß einer bevorzugten Ausführungsform der Erfindung ist das Entleerungsrohr über O-Ring-Dichtungen verschieblich in dem Adapterstück geführt und befestigt. Das Adapterstück kann somit ebenfalls einfach und kostengünstig ausgebildet sein.

Gemäß einer bevorzugten Ausführungsform der Erfindung ist die flexible Umhüllung als ein Schlauch oder eine Schlauchfolie ausgebildet. Dadurch wird eine besonders kostengünstige Sterilbarriere gebildet. Die flexible Umhüllung kann aber auch als ein Faltenbalg ausgebildet sein.

Gemäß einer weiteren bevorzugten Ausführungsform der Erfindung ist das Entleerungsrohr als ein ausziehbares Teleskoprohr ausgebildet. Dadurch wird auch eine Entleerung nahe des Behälterbodens ermöglicht.

Es ist aber auch möglich, das Entleerungsrohr als einen flexiblen Schlauch auszubilden.

Nach einer weiteren bevorzugten Ausführungsform der Erfindung ist in dem Behälterinnenraum an der Wandung mindestens eine Schlauchführung zur Führung des freien Endes des Entleerungsrohres angeordnet. Die Schlauchführung verhindert, dass der flexible Schlauch seitlich ausgelenkt wird, beispielsweise durch von einem Rührer verursachte Strömungen.

Nach einer weiteren bevorzugten Ausführungsform der Erfindung ist der Entleerungsöffnung ein Filter, zum Beispiel ein Tiefenfilter, vorgelagert. Dadurch können gezielt Partikel zurückgehalten werden.

Gemäß einer weiteren bevorzugten Ausführungsform der Erfindung weist das Entleerungsrohr an seinem der Entleerungsöffnung abgewandten Ende einen Teil eines Sterilverbinders auf. Es ist auch möglich, den vorgenannten Sterilverbinder durch einen verschweißbaren Schlauch zur sterilen Verbindung von zwei Schläuchen zu ersetzen.

Nach einer weiteren bevorzugten Ausführungsform der Erfindung ragt in den Behälterinnenraum ein zweites Entleerungsrohr hinein. Dabei kann das zweite Entleerungsrohr auf der dem ersten Entleerungsrohr gegenüberliegenden Seite des Behälterinnenraumes angeordnet sein.

Gemäß einer weiteren bevorzugten Ausführungsform der Erfindung wird der Behälter mit Entleerungsrohr als Einwegbehälter verwendet.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine schematische Seitenansicht eines Behälters mit flexibler Wandung mit einem höhenverstellbaren Entleerungsrohr, das eine flexible Umhüllung aufweist;
- Figur 2:: eine schematische Seitenansicht im Schnitt eines weiteren Behälters mit flexibler Wandung mit einem Entleerungsrohr, das als ein ausziehbares Teleskoprohr ausgebildet ist, und mit einer flexiblen Umhüllung;
- Figur 3:: eine Seitenansicht im Schnitt eines weiteren Behälters mit flexibler Wandung, der zwei Entleerungsrohre mit jeweils einer flexiblen Umhüllung aufweist;
- Figur 4:: eine Seitenansicht im Schnitt eines weiteren Behälters mit flexibler Wandung, der neben einem im Deckenbereich angeordneten ersten Entleerungsrohr mit flexibler Umhüllung im Bodenbereich ein zweites Entleerungsrohr mit flexibler Umhüllung aufweist;
- Figur 5:: eine Seitenansicht eines weiteren Behälters mit flexibler Wandung, der ein als flexibler Schlauch ausgebildetes Entleerungsrohr mit einer im oberen Teilbereich des Entleerungsrohres angeordneten flexiblen Umhüllung und an der Wandung des Behälters angeordneten Schlauchführungen; und
- Figur 6:: eine Seitenansicht im Schnitt des Behälters von Figur 6 mit teilweise herausgezogenem Entleerungsrohr.

### Beschreibung bevorzugter Ausführungsformen

Ein Behälter 1 mit flexibler Wandung 2, beispielsweise für einen Bioreaktor, besteht im Wesentlichen aus einem Behälterinnenraum 3, einem Entleerungsrohr 4 und einer flexiblen Umhüllung 5.

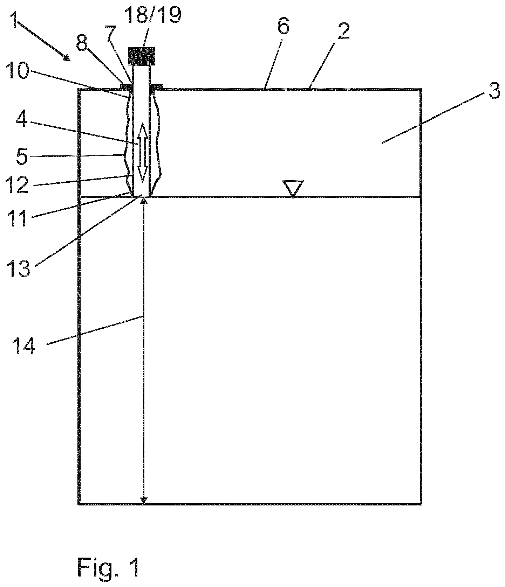

Entsprechend dem Ausführungsbeispiel von Figur 1 weist der Behälter 1 eine in vertikaler Richtung oben angeordnete Decke 6 mit einem Wandungsdurchbruch 7 auf, in dem das längsverschiebliche Entleerungsrohr 4, das in den Behälterinnenraum 3 hineinragt, angeordnet ist. Das Entleerungsrohr 4 kann dabei über Dichtringe in dem Adapterstück 8 geführt werden. Die flexible Umhüllung 5 ist an ihrem zweiten Ende 10 mit dem Adapterstück 8 fluiddicht verbunden, beispielsweise durch Schweißen. An ihrem dem zweiten Ende 10 abgewandten ersten Ende 11 ist die flexible Umhüllung 5 mit dem freien Ende 12 des Entleerungsrohres 4 fluiddicht verbunden, beispielsweise durch Schweißen. Das freie Ende 12 des Entleerungsrohres 4 weist eine Entleerungsöffnung 13 auf, deren Position jeweils die variable Entleerungshöhe 14 bestimmt.

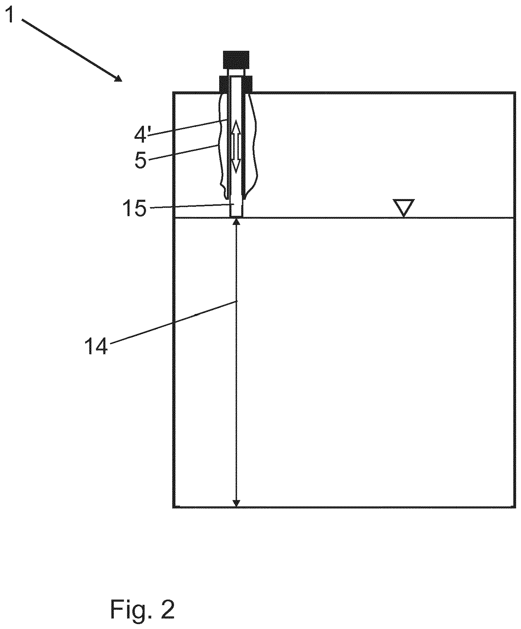

Entsprechend dem Ausführungsbeispiel der Figur 2 ist das Entleerungsrohr 4' als ein ausziehbares Teleskoprohr ausgebildet. Dabei weist das Entleerungsrohr 4' ein zweites ausziehbares oder ausfahrbares Rohr 15 auf.

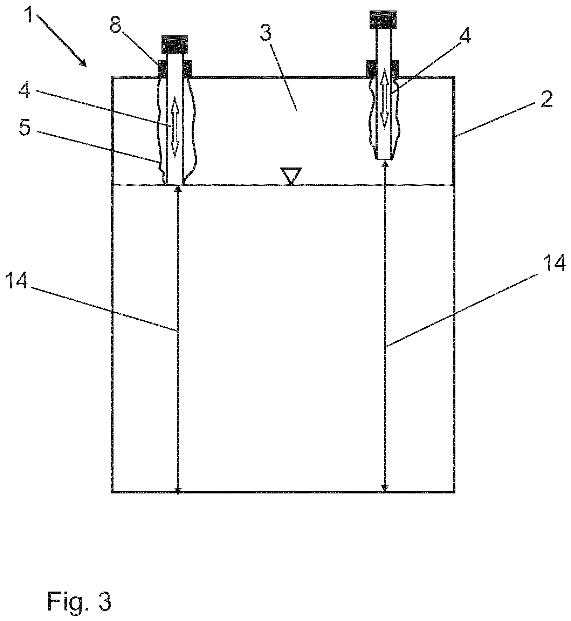

Entsprechend dem Ausführungsbeispiel der Figur 3 weist der Behälter 1 neben dem ersten Entleerungsrohr 4 an der Decke 6 noch ein zweites Entleerungsrohr 4 auf. Das zweite Entleerungsrohr 4 kann aber auch im Bereich des der Decke 6 abgewandten Bodens 16 angeordnet sein, so dass das zweite Entleerungsrohr 4 auf der dem ersten Entleerungsrohr 4 gegenüberliegenden Seite des Behälterinnenraumes 3 angeordnet ist.

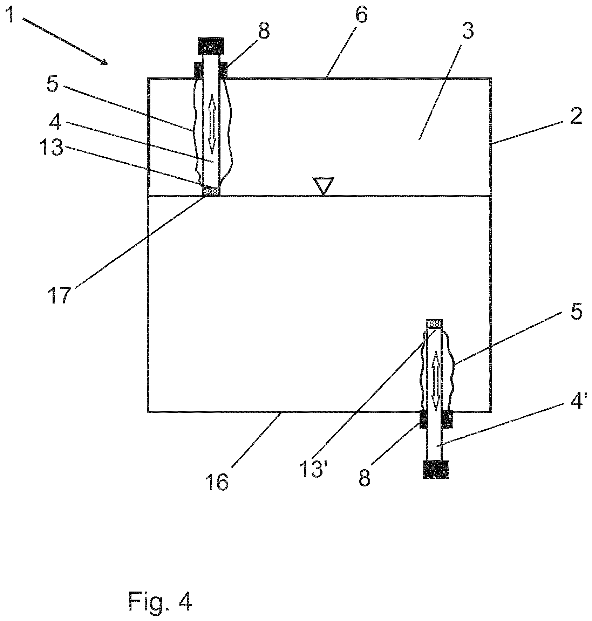

Entsprechend dem Ausführungsbeispiel der Figur 4 kann den Entleerungsrohren 4, 4' an ihren Entleerungsöffnungen 13, 13' an den freien Enden 12 jeweils ein Filter 17 vorlagert sein.

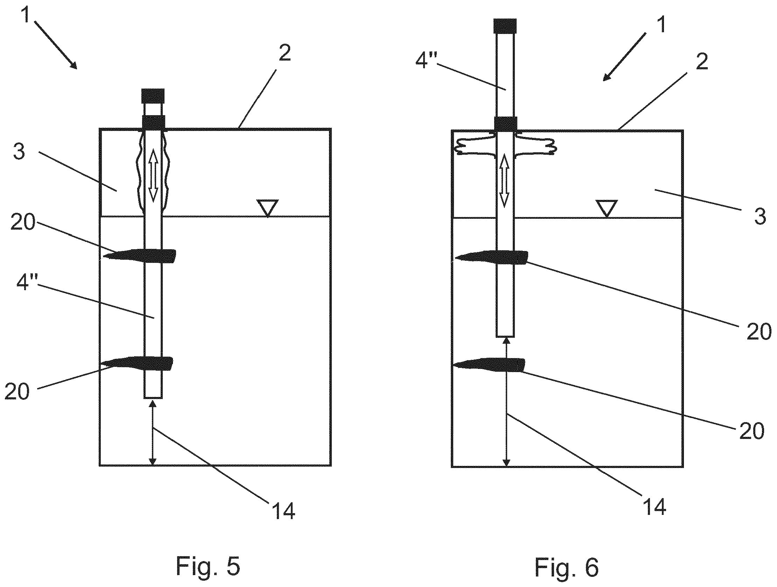

Entsprechend den Ausführungsbeispielen der Figur 5 und 6 kann das Entleerungsrohr 4" als flexibler Schlauch ausgebildet sein, wobei im Behälterinnenraum 3 an der Wandung 2 zwei Schlauchführungen 20 zur Führung des freien Endes des Entleerungsrohres 4" angeordnet sind. Die flexible Umhüllung 5 ist als ein relativ dünner Schlauch oder eine umhüllende Schlauchfolie ausgebildet. Auch kann die Umhüllung 5 als ein Faltenbalg ausgebildet sein.

Der Behälter 1 ist zur Verwendung als Einwegbehälter (single use) vorgesehen und kann insbesondere als Behälter mit flexibler Wandung eines nicht weiter dargestellten Bioreaktors verwendet werden.

Das Entleerungsrohr 4 weist an seinem der Entleerungsöffnung 13 abgewandten Ende einen ersten Teil 18 eines zweiteiligen Sterilverbinders 19 auf.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

### Bezugszeichenliste

- 1: Behälter
- 2: flexible Wandung
- 3: Behälterinnenraum
- 4, 4', 4": Entleerungsrohr
- 5: flexible Umhüllung
- 6: Decke von 1
- 7: Wandungsdurchbruch
- 8: Adapterstück
- 10: zweites Ende von 5
- 11: erstes Ende von 5
- 12: freies Ende von 4
- 13, 13': Entleerungsöffnung
- 14: Entleerungshöhe
- 15: zweites Rohr von 4'
- 16: Boden von 1
- 17: Filter
- 18: erstes Teil von 19
- 19: Sterilverbinder
- 20: Schlauchführung

## Patentansprüche

1. Behälter (1) mit flexibler Wandung (2) und mindestens einem durch einen Wandungsdurchbruch (7) in den von der flexiblen Wandung (2) umgebenen Behälterinnenraum (3) hineinragenden Entleerungsrohr (4, 4', 4"), dessen freies Ende (12) eine Entleerungsöffnung (13) aufweist, die in ihrer Lage auf unterschiedliche Entleerungshöhen (14) in vertikaler Richtung einstellbar ist,
**dadurch gekennzeichnet,**
**dass** in dem Wandungsdurchbruch (7) ein Adapterstück (8) angeordnet ist, in dem das Entleerungsrohr (4, 4', 4") längsverschieblich gehaltert ist,
**dass** das Entleerungsrohr (4, 4', 4") in einem Teilbereich von einer flexiblen Umhüllung (5) umgeben ist, die an einem ersten Ende (11) mit dem Entleerungsrohr (4, 4', 4") und an ihrem dem ersten Ende (11) abgewandten zweiten Ende (10) mit der Wandung (2) oder dem Adapterstück (8) fluiddicht verbunden ist, und
**dass** die flexible Umhüllung (5) im Behälterinnenraum (3) angeordnet ist.

2. Behälter nach Anspruch 1,
**dadurch gekennzeichnet,**
**dass** das Entleerungsrohr (4, 4', 4") über O-Ring-Dichtungen verschieblich in dem Adapterstück (8) geführt und befestigt ist.

3. Behälter nach Anspruch 1 oder 2,
**dadurch gekennzeichnet,**
**dass** die flexible Umhüllung (5) als ein Schlauch oder eine Schlauchfolie ausgebildet ist.

4. Behälter nach Anspruch 1 oder 2,
**dadurch gekennzeichnet,**
**dass** die flexible Umhüllung (5) als ein Faltenbalg ausgebildet ist.

5. Behälter nach einem der Ansprüche 1 bis 4,
**dadurch gekennzeichnet,**
**dass** das Entleerungsrohr (4') als ein ausziehbares Teleskoprohr ausgebildet ist.

6. Behälter nach einem der Ansprüche 1 bis 4,
**dadurch gekennzeichnet,**
**dass** das Entleerungsrohr (4, 4', 4") als ein flexibler Schlauch ausgebildet ist.

7. Behälter nach Anspruch 6,
**dadurch gekennzeichnet,**
**dass** in dem Behälterinnenraum (3) an der Wandung (2) mindestens eine Schlauchführung (20) zur Führung des freien Endes (12) des Entleerungsrohres (4, 4', 4") angeordnet ist.

8. Behälter nach einem der Ansprüche 1 bis 7,
**dadurch gekennzeichnet,**
**dass** der Entleerungsöffnung (13) ein Filter (17) vorgelagert ist.

9. Behälter nach einem der Ansprüche 1 bis 8,
**dadurch gekennzeichnet,**
**dass** das Entleerungsrohr (4, 4', 4") an seinem der Entleerungsöffnung (13) abgewandten Ende einen Teil (18) eines Sterilverbinders (19) aufweist.

10. Behälter nach einem der Ansprüche 1 bis 9,
**dadurch gekennzeichnet,**
**dass** in den Behälterinnenraum (3) ein zweites Entleerungsrohr (4, 4', 4") hineinragt.

11. Behälter nach Anspruch 10,
**dadurch gekennzeichnet,**
**dass** das zweite Entleerungsrohr (4, 4', 4") auf der dem ersten Entleerungsrohr (4, 4', 4") gegenüberliegenden Seite des Behälterinnenraumes (3) angeordnet ist.

12. Behälter nach einem der Ansprüche 1 bis 11,
**dadurch gekennzeichnet,**
**dass** der Behälter (1) mit Entleerungsrohr (4, 4', 4") als Einwegbehälter verwendet wird.

## Claims

1. A container (1) with a flexible wall (2) and with at least one emptying tube (4, 4', 4") which projects through a wall aperture (7) into the container interior (3) surrounded by the flexible wall (2), the free end (12) of which emptying tube has an emptying opening (13) which can be set in its position in the vertical direction to different emptying heights (14)
**characterized in that**
an adapter piece (8), in which the emptying tube (4, 4', 4") is held in a longitudinally displaceable manner, is arranged in the wall aperture (7) and **in that** a portion of the emptying tube (4, 4', 4") is surrounded by a flexible sheath (5) which is connected in a fluid-tight manner at a first end (11) to the emptying tube (4, 4', 4") and at its second end (10), which is remote from the first end (11), to the wall (2) or the adapter piece (8), and
the flexible sheath (5) is arranged within the container interior (3).

2. The container according to Claim 1,
**characterized in that**
the emptying tube (4, 4',4") is positioned and fastened in the adapter piece (8) by means of O ring seals in such a manner that it remains displaceable.

3. The container according to Claim 1 or 2,
**characterized in that**
the flexible sheath (5) is designed as a hose or tubular film.

4. The container according to Claim 1 or 2,
**characterized in that**
the flexible sheath (5) is designed as a bellows hose.

5. The container according to any of Claims 1 to 4,
**characterized in that**
the emptying tube (4') is designed as a extensible telescopic tube.

6. The container according to any of Claims 1 to 4,
**characterized in that**
the emptying tube (4, 4', 4") is designed as a flexible hose.

7. The container according to Claim 6,
**characterized in that**
one or more hose guides (20) are arranged on the wall (2) within the container interior (3) to guide the free end (12) of the emptying tube (4, 4', 4").

8. The container according to any of Claims 1 to 7,
**characterized in that**
a filter (17) is arranged upstream from the emptying opening (13).

9. The container according to any of Claims 1 to 8,
**characterized in that**
the end of the emptying tube (4, 4', 4") facing away from the emptying opening (13) has one part (18) of a sterile connector (19).

10. The container according to any of Claims 1 to 9,
**characterized in that**
a second emptying tube (4, 4', 4") projects into the container interior (3).

11. The container according to Claim 10,
**characterized in that**
the second emptying tube (4, 4', 4") is arranged on the opposite side of the container interior (3) from the first emptying tube (4, 4', 4").

12. The container according to any of Claims 1 to 11,
**characterized in that**
the container (1) with the emptying tube (4, 4', 4") is employed as a single-use container.

## Revendications

1. Conteneur (1), avec une paroi flexible (2) et avec au moins un tube de vidange (4, 4', 4") s'enfonçant par un percement (7) de la paroi dans l'espace intérieur (3) du conteneur entouré par la paroi flexible (2), tube dont l'extrémité libre (12) présente une ouverture de vidange (13) dont la position peut être réglée en direction verticale à différentes hauteurs de vidange (14).
**caractérisé en ce qu'**un adaptateur (8), dans lequel le tube de vidange (4, 4', 4") est maintenu à translation longitudinale, est disposé dans le percement de paroi (7),
**en ce que** le tube de vidange (4, 4', 4") est, dans une région partielle, entouré par une enveloppe flexible (5) qui est reliée en étanchéité aux fluides à une première extrémité (11) au tube de vidange (4, 4', 4"), et à sa seconde extrémité (10) opposée à la première extrémité (11), à la paroi (2) ou à l'adaptateur (8),
et **en ce que** l'enveloppe flexible (5) est disposée dans l'espace intérieur (3) du conteneur.

2. Conteneur selon la revendication 1, **caractérisé en ce que** le tube de vidange (4, 4', 4") est guidé et fixé à translation dans l'adaptateur (8) au moyen de joints toriques d'étanchéité.

3. Conteneur selon la revendication 1 ou 2, **caractérisé en ce que** l'enveloppe flexible (5) est réalisée sous forme de tuyau souple ou de film en gaine.

4. Conteneur selon la revendication 1 ou 2, **caractérisé en ce que** l'enveloppe flexible (5) est réalisée sous forme de soufflet.

5. Conteneur selon l'une des revendications 1 à 4, **caractérisé en ce que** le tube de vidange (4') est réalisé sous la forme d'un tube télescopique déployable.

6. Conteneur selon l'une des revendications 1 à 4, **caractérisé en ce que** le tube de vidange (4, 4', 4") est réalisé sous la forme d'un tuyau flexible.

7. Conteneur selon la revendication 6, **caractérisé en ce qu'**au moins un guide de tuyau (20) est disposé sur la paroi (2) dans l'espace intérieur (3) du conteneur afin de guider l'extrémité libre (12) du tube de vidange (4, 4', 4").

8. Conteneur selon l'une des revendications 1 à 7, **caractérisé en ce qu'**un filtre (17) est monté en amont de l'ouverture de vidange (13).

9. Conteneur selon l'une des revendications 1 à 8, **caractérisé en ce que** le tube de vidange (4, 4', 4") présente, à son extrémité opposée à l'ouverture de vidange (13), une partie (18) d'un connecteur stérile (19).

10. Conteneur selon l'une des revendications 1 à 9, **caractérisé en ce qu'**un deuxième tube de vidange (4, 4', 4") s'enfonce dans l'espace intérieur (3) du conteneur.

11. Conteneur selon la revendication 10, **caractérisé en ce que** le deuxième tube de vidange (4, 4', 4") est disposé sur le côté de l'espace intérieur (3) du conteneur qui fait face au premier tube de vidange (4, 4', 4").

12. Conteneur selon l'une des revendications 1 à 11, **caractérisé en ce que** le conteneur (1) pourvu du tube de vidange (4, 4', 4") est utilisé en tant que récipient à usage unique.
